# EUROPÄISCHE PATENTSCHRIFT

(11) **EP 0 636 617 B1**
(45) Veröffentlichungstag und Bekanntmachung des Hinweises auf die Patenterteilung: **19.05.1999**
(21) Anmeldenummer: 94111340.9
(22) Anmeldetag: 21.07.1994
(51) Int. Cl.: C07D 295/08

(54) **Verfahren zur Herstellung von N-(2-Hydroxyethyl)-piperazin**
Process for the preparation of N-(2-hydroxyethyl)piperazin
Procédé pour la préparation de N-(2-hydroxyéthyle)pipérazine

(30) Priorität: 31.07.1993 DE 4325848
(43) Veröffentlichungstag der Anmeldung: 01.02.1995
(73) Patentinhaber: BASF Aktiengesellschaft, 67063 Ludwigshafen (DE)
(72) Erfinder: Huellmann, Michael, Dr., D-64646 Heppenheim (DE); Becker, Rainer, Dr., D-67098 Bad Duerkheim (DE); Scharf, Emil, Dr., D-67067 Ludwigshafen (DE)

(56) Entgegenhaltungen:
- US-A- 4 328 370
- US-A- 4 338 443
- US-A- 4 725 681

## Beschreibung

Die vorliegende Erfindung betrifft ein Verfahren zur Herstellung von N-(2-Hydroxyethyl)-piperazin durch aminierende Hydrierung von Triethanolamin in der Flüssig- oder Gasphase bei erhöhtem Druck und erhöhter Temperatur in Gegenwart eines Katalysators, der ZrO₂ und/oder Al₂O₃, CuO, CoO und/oder NiO und/oder MoO₃ enthält.

Aus der FR-A-13 81 243 ist die Umsetzung von Triethanolamin mit Ammoniak in Gegenwart von Wasserstoff zu einem Gemisch aus Piperazin (unsubstituiert) und 1,4-Diazabicyclo-(2,2,2)-octan (DABCO) bekannt.

Aus der US-A-4 328 370 ist die Aminierung von Triethanolamin in einer Wasserstoffatmosphäre an Palladiumkatalysatoren zu Gemischen aus Mono- und Diethanolaminverbindungen bekannt.

N,N'-Bis-(2-Hydroxyethyl)-piperazin erhält man nach der DD-A-249 017 in einer diskontinuierlichen Druckreaktion aus Triethanolamin und Ammoniak an vernetzten Polyamiden als Katalysatoren.

Aus der US-A-4 725 681 ist die Umsetzung von Ethanolaminen zu 1,4-Bis-(2-hydroxyethyl)-piperazin und/oder DABCO bekannt.

Aus der US-A-2 541 260 ist weiterhin bekannt, daß man Piperazin mit Ethylenoxid zu einem Gemisch aus N-(2-Hydroxyethyl)-piperazin und N,N'-Bis-(2-Hydroxyethyl)-piperazin umsetzen kann.

Ferner ist aus US-A-4 338 443 die Synthese von N-(2-Hydroxyethyl)-piperazin aus Diethanolamin und Ethanolamin an Nickel/ Kupfer/Chrom-Katalysatoren bekannt, wobei die erzielte Selektivität völlig unbefriedigend ist.

Die bekannten Verfahren lassen wegen der geringen Selektivität bei der Herstellung von N-(2-Hydroxyethyl)-piperazin zu wünschen übrig.

Der vorliegenden Erfindung lag daher die Aufgabe zugrunde, mittels eines alternativen Herstellverfahrens für N-(2-Hydroxyethyl)-piperazin, den zuvor genannten Nachteilen abzuhelfen.

Demgemäß wurde ein neues Verfahren zur Herstellung von N-(2-Hydroxyethyl)-piperazin der Formel I 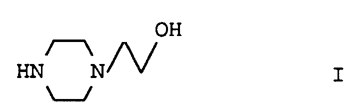 gefunden, welches dadurch gekennzeichnet ist, daß man Triethanolamin der Formel II 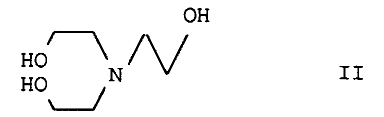 mit Ammoniak in Gegenwart von Wasserstoff bei Temperaturen von 100 bis 500°C und Drücken von 10 bis 500 bar (1 bis 50 MPa) an Heterogenkatalysatoren mit einer aktiven Masse enthaltend

| | |
|---|---|
| 20 bis 85 Gew.-% | ZrO₂ und/oder Al₂O₃, |
| | |
| 1 bis 30 Gew.-% | sauerstoffhaltige Verbindungen des Kupfers, berechnet als CuO, |
| | |
| 1 bis 60 Gew.-% | sauerstoffhaltige Verbindungen des Cobalts berechnet als CoO und/oder sauerstoffhaltige Verbindungen des Nickels, berechnet als NiO und/oder sauerstoffhaltige Verbindungen des Molybdäns, berechnet als MoO₃, |

umsetzt.

Das erfindungsgemäße Verfahren läßt sich wie folgt durchführen: Triethanolamin (II) und Ammoniak können in Gegenwart von Wasserstoff bei Temperaturen von 100 bis 500°C, bevorzugt 150 bis 300°C, besonders bevorzugt 180 bis 250°C und Drucken von 10 bis 500 bar (1 bis 50 MPa), bevorzugt 30 bis 400 bar (3 bis 40 MPa), besonders bevorzugt 35 bis 300 bar (3,5 bis 30 MPa) mit Heterogenkatalysatoren umgesetzt werden.

Als Heterogenkatalysatoren eignen sich solche, die als katalytisch aktive Masse 20 bis 85 Gew.-%, bevorzugt 25 bis 80 Gew.%, besonders bevorzugt 30 bis 75 Gew.% ZrO₂ und/oder Al₂O₃, 1 bis 30 Gew.-%, bevorzugt 2 bis 25 Gew.%, besonders bevorzugt 4 bis 20 Gew.% sauerstoffhaltige Verbindungen des Kupfers wie Kupfer-I-oxid und Kupfer-II-oxid, bevorzugt Kupfer-I-oxid, besonders bevorzugt Kupfer-II-oxid, berechnet als CuO, 1 bis 60 Gew.-%, bevorzugt 1 bis 30 Gew.%, besonders bevorzugt 1 bis 20 Gew.% sauerstoffhaltige Verbindungen des Cobalts wie Cobalt-I-oxid und Cobalt-II-xoid, bevorzugt Cobalt-I-oxid, besonders bevorzugt Cobalt-II-oxid, berechnet als CoO und/oder sauerstoffhaltige Verbindungen des Nickels wie Nickel-I-oxid und Nickel-II-oxid, bevorzugt Nickel-I-oxid, besonders bevorzugt Nickel-II-oxid, berechnet als NiO und/oder sauerstoffhaltige Verbindungen des Molybdäns, bevorzugt Molybdän-V-oxid, besonders bevorzugt Molybdän-VI-oxid, berechnet als MoO₃, enthalten.

Ammoniak wird in der Regel gasförmig oder flüssig, im allgemeinen jedoch nicht in wäßriger Lösung, im Molverhältnis von Ammoniak zum Triethanolamin (II) von 1 : 1 bis 50 : 1, bevorzugt 1,5 : 1 bis 30 : 1, besonders bevorzugt 2 : 1 bis 20 : 1 eingesetzt. Der Ammoniaküberschuß kann ohne weiteres auch größer als 50 : 1 sein.

Der Wasserstoff wird der Reaktion im allgemeinen in einer Menge von 5 bis 400 1, bevorzugt in einer Menge von 50 bis 200 1 pro Mol Triethanolamin zugeführt, wobei die Literangaben jeweils auf Normalbedingungen umgerechnet wurden (S.T.P.).

Praktisch geht man bei der Durchführung der Umsetzung so vor, daß man dem Heterogenkatalysator, der sich üblicherweise in einem bevorzugt von außen beheizten Festbettreaktor befindet, bei der gewünschten Reaktionstemperatur und dem gewünschten Druck das Triethanolamin und Ammoniak simultan zuführt. Dabei belastet man den Heterogenkatalysator im allgemeinen mit 0,01 bis 5,0, bevorzugt 0,1 bis 2,0 und insbesondere bevorzugt mit 0,2 bis 1,5 1 Triethanolamin pro Liter Heterogenkatalysator und Stunde.

Hierbei ist es zweckmäßig, die Reaktanten bereits vor der Zuführung in das Reaktionsgefäß zu erwärmen, und zwar bevorzugt auf die Reaktionstemperatur.

Der Reaktor kann sowohl in der Sumpf- als auch in der Rieselfahrweise betrieben werden, d. h. man kann die Reaktanten sowohl von unten nach oben als auch von oben nach unten durch den Reaktor leiten.

Die Reaktion kann sowohl diskontinuierlich als auch kontinuierlich durchgeführt werden. In beiden Fällen kann das überschüssige Ammoniak zusammen mit dem Wasserstoff im Kreis geführt werden. Ist der Umsatz bei der Reaktion nicht vollständig, so kann das nicht umgesetzte Ausgangsmaterial ebenfalls in die Reaktionszone zurückgeführt werden.

Aus dem Reaktionsaustrag können, nachdem dieser zweckmäßigerweise entspannt worden ist, der überschüssige Ammoniak und der Wasserstoff entfernt und das erhaltene Cyclisierungsprodukt durch Destillation gereinigt werden. Ammoniak und Wasserstoff können vorteilhaft wieder in die Reaktionszone zurückgeführt werden. Das gleiche gilt für das eventuell nicht vollständig umgesetzte Triethanolamin.

Das im Zuge der Umsetzung gebildete Reaktionswasser wirkt sich im allgemeinen auf den Umsetzungsgrad, die Reaktionsgeschwindigkeit, die Selektivität und die Katalysatorstandzeit nicht störend aus und kann deshalb zweckmäßigerweise erst bei der destillativen Aufarbeitung des Reaktionsproduktes aus diesem entfernt werden. Das N-(2-Hydroxyethyl)-piperazin (I) findet z.B. als Zwischenprodukte für die Synthese von Wirkstoffen, Pflanzenschutzmitteln und insbesondere zur Herstellung von 1,4-Diazabicyclo-(2,2,2)-octan (DABCO), einem Katalysator zur Herstellung von Polyurethanen (US-A-3,166,558) Verwendung.

### Beispiel

Triethanolamin wird zusammen mit NH₃ und H₂ über einen Vorheizer zu dem in Sumpffahrweise betriebenen Hochdruckreaktor, der eine Innentemperatur von 220 bis 230°C hat, gefahren. Man fährt zur Entfernung von Inerten zweckmäßig ein leichtes Abgas (50 l/h/1 1 Kontakt).

Der Reaktor ist mit einem Katalysator der Zusammensetzung 10 % CoO, 10 % NiO, 4 % CuO auf ZrO₂-Träger, reduziert mit Wasserstoff (bei zuletzt 400°C), gefüllt. Das Molverhältnis Triethanolamin : Ammoniak beträgt 1 : 1,3, die Katalysatorbelastung 1,485 kg Triethanolamin und 0,22 kg Ammoniak pro 1 Kontakt/ Stunde.

Der Ofenaustrag wird 2-stufig in einem Mitteldruckabscheider entspannt und destillativ aufgearbeitet. Überschüssiges NH₃ und nichtumgesetztes Triethanolamin (25 %) werden nach der destillativen Abtrennung zurückgeführt.

GC-Analyse des Aminierungsaustrags (ammoniak- bzw. wasserfrei) [Flächen-%] :

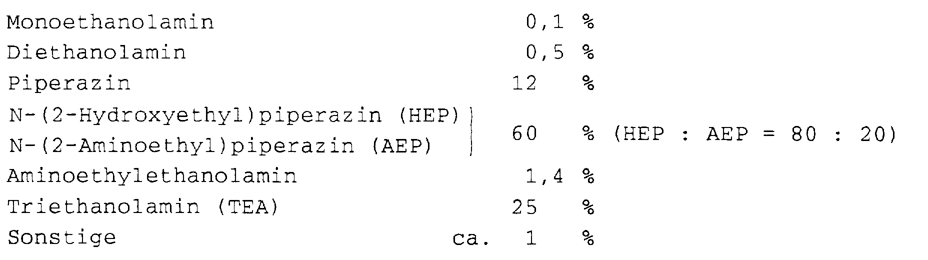

Die Reaktion ist bisher kontinuierlich durchgeführt worden. Die Katalysatorstandzeiten sind befriedigend. So wurde bei der beschriebenen Einstellung (Triethanolamin/Ammoniak = 1 : 1,3 / 220°C, 200 bar H₂) z.B. eine Standzeit von 4 Monaten ohne drastischen Aktivitätsverlust (minus 10 bis 20 %) und ohne HEP-Selektivitätsverlust beobachtet.

Das Verhältnis HEP/AEP kann schwanken. Erniedrigung des Triethanolamin/ Ammoniak-Verhältnis z.B. auf 1 : 10 ergibt bereits HEP : AEP = 20 : 80.

## Patentansprüche

1. Verfahren zur Herstellung von N-(2-Hydroxyethyl)-piperazin der Formel I 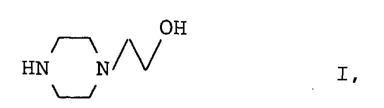 dadurch gekennzeichnet, daß man Triethanolamin der Formel II 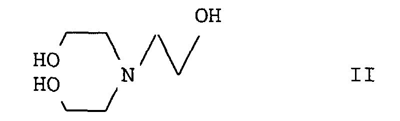 mit Ammoniak bei einem Molverhältnis von Ammoniak zu Triethanolamin von 1:1 bis 50:1 in Gegenwart von Wasserstoff bei Temperaturen von 100 bis 500°C und Drücken von 10 bis 500 bar (1 bis 50 MPa) an Heterogenkatalysatoren mit einer aktiven Masse enthaltend
| | |
|---|---|
| 20 bis 85 Gew.-% | ZrO₂ und/oder Al₂O₃, |
| | |
| 1 bis 30 Gew.-% | sauerstoffhaltige Verbindungen des Kupfers, berechnet als CuO, |
| | |
| 1 bis 60 Gew.-% | sauerstoffhaltige Verbindungen des Cobalts berechnet als CoO und/oder sauerstoffhaltige Verbindungen des Nickels, berechnet als NiO und/oder sauerstoffhaltige Verbindungen des Molybdäns, berechnet als MoO₃, |
umsetzt.

2. Verfahren zur Herstellung von N-(2-Hydroxyethyl)-piperazin der Formel I nach Anspruch 1, dadurch gekennzeichnet, daß man die Umsetzung bei Temperaturen von 150 bis 300°C durchführt.

3. Verfahren zur Herstellung von N-(2-Hydroxyethyl)-piperazin der Formel I nach Anspruch 1, dadurch gekennzeichnet, daß man die Umsetzung bei Temperaturen von 180 bis 250°C durchführt.

4. Verfahren zur Herstellung von N-(2-Hydroxyethyl)-piperazin der Formel I nach Anspruch 1, dadurch gekennzeichnet, daß man die Umsetzung bei Drücken von 30 bis 400 bar (3 bis 40 MPa) durchführt.

5. Verfahren zur Herstellung von N-(2-Hydroxyethyl)-piperazin der Formel I nach Anspruch 1, dadurch gekennzeichnet, daß man die Umsetzung bei Drücken von 35 bis 300 bar (3,5 bis 30 MPa) durchführt.

6. Verfahren zur Herstellung von N-(2-Hydroxyethyl)-piperazin der Formel I nach Anspruch 1, dadurch gekennzeichnet, daß man die Umsetzung mit einem Molverhältnis von Ammoniak zum Triethanolamin von 1 : 1 bis 50 : 1 durchführt.

7. Verfahren zur Herstellung von N-(2-Hydroxyethyl)-piperazin der Formel I nach Anspruch 1, dadurch gekennzeichnet, daß man die Umsetzung in Gegenwart von Heterogenkatalysatoren mit einer aktiven Masse enthaltend
| | |
|---|---|
| 25 bis 80 Gew.-% | ZrO₂ und/oder Al₂O₃, |
| | |
| 2 bis 25 Gew.-% | sauerstoffhaltige Verbindungen des Kupfers, berechnet als CuO, |
| | |
| 1 bis 30 Gew.-% | sauerstoffhaltige Verbindungen des Cobalts, berechnet als CoO und/oder sauerstoffhaltige Verbindungen des Nickels, berechnet als NiO und/oder sauerstoffhaltige Verbindungen des Molybdäns, berechnet als MoO₃, |
durchführt.

## Claims

1. A process for the preparation of N-(2-hydroxyethyl)-piperazine of the formula I 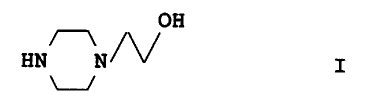 which comprises reacting triethanolamine of the formula II 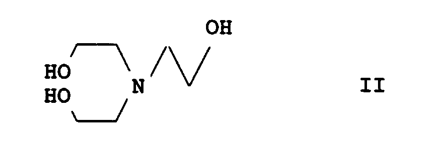 with ammonia at a molar ratio of ammonia to triethanolamine of from 1:1 to 50:1 in the presence of hydrogen at from 100 to 500°C and from 10 to 500 bar (1 to 50 MPa) over a heterogeneous catalyst having an active material containing
| | |
|---|---|
| from 20 to 85 % | by weight of ZrO₂ or Al₂O₃, |
| | |
| from 1 to 30 % | by weight of oxygen-containing compounds of copper, calculated as CuO, and |
| | |
| from 1 to 60 % | by weight of oxygen-containing compounds of cobalt, calculated as CoO, or oxygen-containing compounds of nickel, calculated as NiO, or oxygen-containing compounds of molybdenum, calculated as MoO₃. |

2. A process for the preparation of N-(2-hydroxyethyl)-piperazine of the formula I as claimed in claim 1, wherein the reaction is carried out at from 150 to 300°C.

3. A process for the preparation of N-(2-hydroxyethyl)-piperazine of the formula I as claimed in claim 1, wherein the reaction is carried out at from 180 to 250°C.

4. A process for the preparation of N-(2-hydroxyethyl)-piperazine of the formula I as claimed in claim 1, wherein the reaction is carried out at from 30 to 400 bar (3 to 40 MPa).

5. A process for the preparation of N-(2-hydroxyethyl)-piperazine of the formula I as claimed in claim 1, wherein the reaction is carried out at from 35 to 300 bar (3.5 to 30 MPa).

6. A process for the preparation of N-(2-hydroxyethyl)-piperazine of the formula I as claimed in claim 1, wherein the reaction is carried out using a molar ratio of ammonia to triethanolamine of from 1:1 to 50:1.

7. A process for the preparation of N-(2-hydroxyethyl)-piperazine of the formula I as claimed in claim 1, wherein the reaction is carried out in the presence of a heterogeneous catalyst having an active material containing
| | |
|---|---|
| from 25 to 80 % | by weight of ZrO₂ or Al₂O₃, |
| | |
| from 2 to 25 % | by weight of oxygen-containing compounds of copper, calculated as CuO, and |
| | |
| from 1 to 30 % | by weight of oxygen-containing compounds of cobalt, calculated as CoO, or oxygen-containing compounds of nickel, calculated as NiO, or oxygen-containing compounds of molybdenum, calculated as MoO₃. |

## Revendications

1. Procédé pour préparer la N-(2-hydroxyéthyl)-pipérazine de formule I 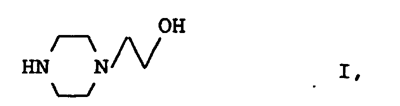 caractérisé en ce qu'on fait réagir une triéthanolamine de formule II 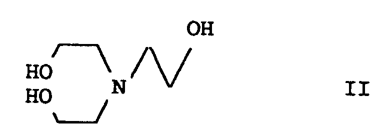 avec de l'ammoniac selon un rapport en moles de l'ammoniac à la triéthanolamine de 1:1 à 50:1, en présence d'hydrogène à des températures de 100 à 500°C et sous des pressions de 10 à 500 bar (1 à 50 MPa), sur des catalyseurs hétérogènes ayant une masse active contenant :
| | |
|---|---|
| de 20 à 85 % en poids | de ZrO₂ et/ou d'Al₂O₃, |
| de 1 à 30 % en poids | de composés oxygénés du cuivre, exprimés en CuO, |
| de 1 à 60 % en poids | de composés oxygénés du cobalt, exprimés en CoO, et/ou de composés oxygénés du nickel, exprimés en NiO, et/ou de composés oxygénés du molybdène, calculés en MoO₃. |

2. Procédé pour préparer la N-(2-hydroxyéthyl)-pipérazine de formule I selon la revendication 1, caractérisé en ce qu'on met en oeuvre la réaction à des températures de 150 à 300°C.

3. Procédé pour préparer la N-(2-hydroxyéthyl)-pipérazine de formule I selon la revendication 1, caractérisé en ce qu'on met en oeuvre la réaction à des températures de 180 à 250°C.

4. Procédé pour préparer la N-(2-hydroxyéthyl)-pipérazine de formule I selon la revendication 1, caractérisé en ce qu'on met en oeuvre la réaction sous des pressions de 30 à 400 bar (3 à 40 MPa).

5. Procédé pour préparer la N-(2-hydroxyéthyl)-pipérazine de formule I selon la revendication 1, caractérisé en ce qu'on met en oeuvre la réaction sous des pressions de 35 à 300 bar (3,5 à 30 MPa).

6. Procédé pour préparer la N-(2-hydroxyéthyl)-pipérazine de formule I selon la revendication 1, caractérisé en ce qu'on met en oeuvre la réaction avec un rapport en moles de l'ammoniac à la triéthanolamine de 1:1 à 50:1.

7. Procédé pour préparer la N-(2-hydroxyéthyl)-pipérazine de formule I selon la revendication 1, caractérisé en ce qu'on met en oeuvre la réaction en présence de catalyseurs hétérogènes ayant une masse active contenant :
| | |
|---|---|
| de 25 à 80 % en poids | de ZrO₂ et/ou d'Al₂O₃, |
| de 2 à 25 % en poids | de composés oxygénés du cuivre, exprimés en CuO, |
| de 1 à 30 % en poids | de composés oxygénés du cobalt, exprimés en CoO, et/ou de composés oxygénés du nickel, exprimés en NiO, et/ou de composés oxygénés du molybdène, calculés en MoO₃. |
